(19) Europäisches Patentamt
European Patent Office
Office européen des brevets

(11) **EP 2 831 562 B1**

(12) **FASCICULE DE BREVET EUROPEEN**

(45) Date de publication et mention
de la délivrance du brevet:
**30.11.2016 Bulletin 2016/48**

(21) Numéro de dépôt: **13722483.8**

(22) Date de dépôt: **29.03.2013**

(51) Int Cl.:
*G01N 3/28* (2006.01)   *G01N 11/16* (2006.01)
*G01N 33/38* (2006.01)   *G01N 11/00* (2006.01)
*B28C 7/02* (2006.01)   *B28C 5/00* (2006.01)
*G01N 11/14* (2006.01)

(86) Numéro de dépôt international:
**PCT/FR2013/050711**

(87) Numéro de publication internationale:
**WO 2013/144528 (03.10.2013 Gazette 2013/40)**

(54) **PROCÈDE DE CONTRÔLE D'UN PARAMÈTRE D'OUVRABILITE D'UN BÉTON DANS UN MALAXEUR**

VERFAHREN ZUR STEUERUNG EINER VERARBEITBARKEIT IN EINEM BETONMISCHER

METHOD FOR CONTROLLING AN WORKABILITY PARAMETER IN A CONCRETE MIXER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.03.2012 FR 1252938**

(43) Date de publication de la demande:
**04.02.2015 Bulletin 2015/06**

(73) Titulaire: **Lafarge**
**75116 Paris (FR)**

(72) Inventeurs:
• **ROY, Cédric**
**F-38300 Crachier (FR)**
• **LOMBOIS-BURGER, Hélène**
**F-69003 Lyon (FR)**
• **BLACHIER, Christian**
**F-38200 Vienne (FR)**
• **JUGE, Cédric**
**F-38080 L'isle D'abeau (FR)**
• **TOUSSAINT, Fabrice**
**F-38080 Saint-alban De Roche (FR)**

(74) Mandataire: **Keschmann, Marc et al**
**Haffner und Keschmann**
**Patentanwälte GmbH**
**Schottengasse 3a**
**1014 Wien (AT)**

(56) Documents cités:
**WO-A1-2011/162878      FR-A1- 2 604 785**
**US-A1- 2004 149 019**

**Description**

Domaine de l'invention

La présente invention concerne un procédé de contrôle d'au moins un paramètre d'ouvrabilité, par exemple l'affaissement, l'étalement, la contrainte seuil, la viscosité ou le temps d'écoulement d'un béton dans le réservoir d'un malaxeur à axe de rotation non-vertical.

Exposé de l'art antérieur

Un béton est un mélange de granulats agglomérés par un liant et de l'eau. Le liant peut être un liant hydraulique, par exemple du ciment. On parle alors de béton de ciment. Le liant peut être un liant hydrocarboné, par exemple du bitume. On parle alors de béton bitumineux.

Lorsqu'il est réalisé, le béton a une consistance plus ou moins fluide, puis il durcit jusqu'à devenir solide. Le béton doit donc être mis en place avant d'avoir trop durci. L'ouvrabilité du béton correspond à la facilité avec laquelle le béton peut être manipulé. L'ouvrabilité d'un béton peut être caractérisée par la mesure de paramètres rhéologiques tels que la contrainte seuil ou la viscosité du béton ou par la mesure de paramètres issus de tests usuels réalisés sur le site d'utilisation du béton, tels que l'affaissement (en anglais slump), l'étalement (en anglais slump flow) ou le temps d'écoulement. A titre d'exemple, l'affaissement peut être mesuré selon le test décrit dans la norme européenne NF EN 12350-2 de décembre 1999.

La mesure des paramètres rhéologiques nécessite généralement des appareils de mesure spécifiques. Il peut être difficile de réaliser ces mesures sur le site d'utilisation du béton. Au contraire, l'affaissement, l'étalement et le temps d'écoulement peuvent être facilement mesurés sur le site d'utilisation du béton.

Toutefois, un besoin existe de pouvoir mesurer le paramètre d'ouvrabilité lorsque le béton se trouve dans un malaxeur à axe de rotation non-vertical et qu'il n'est pas alors possible d'accéder directement au béton afin d'éviter une dérive trop importante du paramètre d'ouvrabilité. C'est le cas, par exemple, lorsque le béton se trouve dans le réservoir d'un camion-toupie lors du transport du béton depuis le site de fabrication du béton jusqu'au site d'utilisation du béton.

Il existe des procédés de mesure indirecte de l'affaissement d'un béton dans un malaxeur. A titre d'exemple, le brevet US 5 713 663 décrit un procédé de mesure indirecte de l'affaissement d'un béton dans le réservoir tournant d'un camion-toupie à partir du couple d'entraînement appliqué au réservoir. L'affaissement peut alors être ajusté par ajout d'eau ou d'adjuvant au béton. Dans le cas où le réservoir est entraîné en rotation par un moteur hydraulique, le couple moteur peut être déterminé à partir de la mesure de la pression du fluide hydraulique fourni au moteur. L'affaissement est alors déterminé par une formule empirique à partir de la pression hydraulique mesurée.

Le procédé comprend une étape préalable de détermination, pour chaque formulation de béton susceptible d'être fabriquée, de la formule empirique représentant l'évolution de l'affaissement du béton en fonction de la pression hydraulique.

Un béton ordinaire correspond à un béton pour lequel l'affaissement est généralement compris entre 10 mm et 220 mm mesuré selon la norme européenne NF EN 12350-2 de décembre 1999. Le test consiste à remplir un tronc de cône de référence avec le béton à tester, à libérer le béton du tronc de cône, puis à déterminer la hauteur de laquelle le béton s'est affaissé.

Un béton fluide est un béton pour lequel l'affaissement est trop important pour être mesuré correctement par le test de la norme européenne NF EN 12350-2 de décembre 1999. Dans ce cas, on peut mesurer l'étalement qui correspond au test précédent à la différence que l'on mesure le diamètre de la galette de béton obtenue après le retrait du moule selon la norme européenne NF EN 12350-8 de novembre 2010. On peut également mesurer le temps d'écoulement selon la norme européenne NF EN 12350-9 de novembre 2010 en faisant s'écouler le béton dans un entonnoir et en mesurant la durée d'écoulement du béton entre deux repères de l'entonnoir.

Le procédé de mesure décrit dans le brevet US 5 713 663 n'est pas adapté aux bétons fluides. En effet, pour les bétons fluides, l'affaissement/l'étalement du béton varie peu en fonction de la pression hydraulique. Il n'est donc pas possible d'obtenir une mesure précise de l'affaissement/l'étalement du béton en mesurant la pression hydraulique selon le procédé du brevet US 5 713 663.

Un autre inconvénient d'un tel procédé de mesure est qu'il est nécessaire de déterminer la formule empirique représentant l'évolution de l'affaissement du béton en fonction de la pression hydraulique pour chaque formulation de béton susceptible d'être fabriquée. De ce fait, le procédé ne peut pas être mis en oeuvre lorsque la formulation du béton est modifiée. Il est alors nécessaire de déterminer une nouvelle formule empirique pour la nouvelle formulation.

Un autre inconvénient d'un tel procédé de mesure est qu'il ne permet pas de mesurer des paramètres d'ouvrabilité du béton autres que l'affaissement, par exemple la contrainte de seuil ou la viscosité du béton. Toutefois, il peut être avantageux de mesurer de tels paramètres rhéologiques dans le cas des bétons fluides qui sont susceptibles d'être pompés.

Il existe donc un besoin d'un procédé de contrôle d'au moins un paramètre d'ouvrabilité, notamment l'affaissement, l'étalement, la contrainte seuil, le temps d'écoulement et/ou la viscosité d'un béton dans le réservoir d'un malaxeur à axe de rotation non-vertical qui permette la détermination avec précision de ce paramètre d'ouvrabilité même dans le cas où la fluidité du béton est importante.

Résumé

Un objet de la présente invention est de pallier tout ou partie des inconvénients décrits précédemment.
Un autre objet de la présente invention est de proposer un procédé de contrôle d'un paramètre d'ouvrabilité, notamment l'affaissement, l'étalement, la contrainte seuil, le temps d'écoulement et/ou la viscosité, d'un béton dans le réservoir d'un malaxeur à axe de rotation non-vertical qui ne dépend pas de la fluidité du béton.
Un autre objet de la présente invention est que le procédé peut être mis en oeuvre pour de nouvelles formulations de béton sans nécessiter d'opérations d'adaptation supplémentaires.
Ainsi, la présente invention prévoit un procédé de contrôle d'au moins un paramètre d'ouvrabilité d'un béton contenu dans le réservoir d'un malaxeur à axe de rotation non-vertical, comprenant les étapes suivantes :

faire tourner le réservoir à au moins deux vitesses de rotation différentes ;
déterminer, pour chacune desdites au moins deux vitesses de rotation $\omega$, un couple d'entraînement C en rotation du réservoir, une valeur de contrainte de cisaillement $\tau$ du béton et une valeur de gradient de vitesse $\dot{\gamma}$ du béton selon les relations suivantes :

$$\tau = T(\omega) \cdot C$$

$$\dot{\gamma} = G(\omega) \cdot \omega$$

où T et G sont des fonctions prédéterminées ;
déterminer une relation d'évolution de la contrainte de cisaillement $\tau$ en fonction du gradient de vitesse $\dot{\gamma}$ par extrapolation et/ou approximation à partir des valeurs déterminées ; et
fournir une indication du paramètre d'ouvrabilité du béton à partir de la relation d'évolution.

Selon un exemple de réalisation de l'invention, le procédé comprend les étapes suivantes :

faire tourner le réservoir à une première vitesse de rotation et déterminer un premier couple d'entraînement en rotation du réservoir à la première vitesse de rotation ;
faire tourner le réservoir à une deuxième vitesse de rotation et déterminer un deuxième couple d'entraînement en rotation du réservoir à la deuxième vitesse de rotation ;
déterminer une première contrainte de cisaillement égale au produit du premier couple et de la valeur de la fonction T à la première vitesse de rotation ;
déterminer un premier gradient de vitesse égal au produit de la première vitesse de rotation et de la valeur de la fonction G à la première vitesse de rotation ;
déterminer une deuxième contrainte de cisaillement égale au produit du deuxième couple et de la valeur de la fonction T à la deuxième vitesse de rotation ;
déterminer un deuxième gradient de vitesse égal au produit de la deuxième vitesse de rotation et de la valeur de la fonction G à la deuxième vitesse de rotation ; et
déterminer la relation d'évolution de la contrainte de cisaillement en fonction du gradient de vitesse par extrapolation et/ou approximation à partir des première et deuxième contraintes de cisaillement et des premier et deuxième gradients de vitesse.

Selon un exemple de réalisation de l'invention, le procédé comprend les étapes suivantes :

faire tourner le réservoir à une troisième vitesse de rotation et déterminer un troisième couple d'entraînement en rotation du réservoir à la troisième vitesse de rotation ;
déterminer une troisième contrainte de cisaillement égale au produit du troisième couple et de la valeur de la fonction T à la troisième vitesse de rotation ;
déterminer un troisième gradient de vitesse égal au produit de la troisième vitesse de rotation et de la valeur de la fonction G à la troisième vitesse de rotation ; et
déterminer la relation d'évolution de la contrainte de cisaillement en fonction du gradient de vitesse par extrapolation
et/ou approximation en outre à partir de la troisième contrainte de cisaillement et du troisième gradient de vitesse.

Selon un exemple de réalisation de l'invention, le paramètre d'ouvrabilité du béton est choisi parmi l'affaissement, l'étalement, la contrainte seuil, la viscosité et le temps d'écoulement.

Selon un exemple de réalisation de l'invention, le procédé comprend la détermination de la contrainte seuil du béton à partir de la relation d'évolution et la détermination de l'affaissement et/ou de l'étalement à partir de la contrainte seuil.

Selon un exemple de réalisation de l'invention, le procédé comprend l'ajustement dans le réservoir du paramètre d'ouvrabilité du béton par l'introduction d'un composé dans le réservoir.

Selon un exemple de réalisation de l'invention, le composé comprend de l'eau, un adjuvant ou un mélange de ceux-ci.

Selon un exemple de réalisation de l'invention, la fourniture de l'indication du paramètre d'ouvrabilité du béton comprend l'affichage sur un écran d'affichage du paramètre d'ouvrabilité, l'impression du paramètre d'ouvrabilité sur un support et/ou le stockage d'une donnée représentative du paramètre d'ouvrabilité dans une mémoire.

Selon un exemple de réalisation de l'invention, le réservoir est entraîné en rotation par un moteur hydraulique comprenant une entrée de réception d'un fluide hydraulique et une sortie de refoulement du fluide hydraulique, le couple étant déterminé à partir d'une première différence de pressions égale à la différence entre la pression hydraulique mesurée à l'entrée du moteur hydraulique et la pression hydraulique mesurée à la sortie du moteur hydraulique.

Selon un exemple de réalisation de l'invention, la première différence de pressions est diminuée d'une deuxième différence de pressions égale à la différence entre la pression hydraulique à l'entrée du moteur hydraulique et la pression hydraulique à la sortie du moteur hydraulique en l'absence de béton dans le réservoir à la vitesse de rotation de la mesure.

Selon un exemple de réalisation de l'invention, la pression hydraulique mesurée à l'entrée ou à la sortie du moteur hydraulique est égale à la moyenne d'un nombre de valeurs de pression échantillonnées, ledit nombre étant inversement proportionnel à la vitesse de rotation du réservoir.

Selon un exemple de réalisation de l'invention, pendant l'échantillonnage des valeurs de pression utilisées pour obtenir la pression hydraulique mesurée à l'entrée ou à la sortie du moteur hydraulique, les variations de la vitesse de rotation du réservoir sont inférieures à un seuil.

Selon un exemple de réalisation de l'invention, les fonctions G et T sont obtenues en déterminant :

pour chaque béton d'une pluralité de bétons différents, une courbe d'évolution du couple d'entraînement du réservoir contenant ledit béton en fonction de la vitesse de rotation du réservoir ;

pour chaque béton de la pluralité de bétons différents, une courbe d'évolution de la contrainte de cisaillement du béton en fonction du gradient de vitesse du béton au moyen d'un rhéomètre ; et

pour chaque paire de bétons de la pluralité de bétons différents, un premier point d'intersection entre les courbes d'évolution du couple d'entraînement du réservoir en fonction de la vitesse de rotation du réservoir pour les bétons de la paire et un deuxième point d'intersection entre les courbes d'évolution de la contrainte de cisaillement en fonction du gradient de vitesse pour les bétons de la paire.

Selon un exemple de réalisation de l'invention, pour le premier point d'intersection et le deuxième point d'intersection de chaque paire de bétons de la pluralité de bétons différents, il est déterminé la valeur $G_i^{CC}$ de la fonction G et la valeur $T_i^{CC}$ de la fonction T selon les relations suivantes :

$$G_i^{CC} = \frac{\gamma_i}{\omega_i}$$

$$T_i^{CC} = \frac{\tau_i}{C_i}$$

où $\dot{\gamma}_i$ est le gradient de vitesse au second point d'intersection, $\tau_i$ est la contrainte de cisaillement du béton au second point d'intersection, $C_i$ est le couple d'entraînement au premier point d'intersection et $\omega_i$ est la vitesse de rotation au premier point d'intersection.

Selon un exemple de réalisation de l'invention, pour le premier point d'intersection et le second point d'intersection de chaque paire de bétons de la pluralité de bétons différents, il est déterminé la valeur $G_i^{Alt}$ de la fonction G et la valeur $T_i^{Alt}$ de la fonction T selon les relations suivantes :

$$G_i^{Alt} = \sqrt{\frac{C_i}{V \cdot \eta_i \cdot \omega_i}}$$

$$T_i^{Alt} = \frac{1}{G_i^{Pow} \cdot V}$$

où V est le volume du béton dans le réservoir, $\eta_i$ est la viscosité apparente du béton égale au rapport entre la contrainte de cisaillement du béton au second point d'intersection et le gradient de vitesse au second point d'intersection, $C_i$ est le couple d'entraînement au premier point d'intersection et $\omega_i$ est la vitesse de rotation au premier point d'intersection.

La présente invention prévoit également un dispositif mémoire sur lequel est stocké un programme d'ordinateur pour la mise en oeuvre du procédé défini précédemment.

La présente invention prévoit également un dispositif de contrôle d'au moins un paramètre d'ouvrabilité d'un béton, comprenant :

un malaxeur à axe de rotation non-vertical comportant un réservoir contenant le béton ;
un système d'entraînement en rotation du réservoir adapté à faire tourner le réservoir à au moins deux vitesses de rotation différentes ;
un premier capteur de mesure d'une donnée représentative du couple d'entraînement en rotation du réservoir ;
un deuxième capteur de mesure d'une donnée représentative de la vitesse de rotation du réservoir ; et
un module de traitement relié au système d'entraînement et aux premier et deuxième capteurs et configuré pour déterminer, pour chacune desdites au moins deux vitesses de rotation $\omega$, un couple d'entraînement C en rotation du réservoir, une valeur de contrainte de cisaillement $\tau$ du béton et une valeur de gradient de vitesse $\dot{\gamma}$ du béton selon les relations suivantes :

$$\tau = T(\omega) \cdot C$$

$$\dot{\gamma} = G(\omega) \cdot \omega$$

où T et G sont des fonctions prédéterminées ;
déterminer une relation d'évolution de la contrainte de cisaillement $\tau$ en fonction du gradient de vitesse $\dot{\gamma}$ par extrapolation et/ou approximation à partir des valeurs déterminées ; et
fournir une indication du paramètre d'ouvrabilité du béton à partir de la relation d'évolution.

Brève description des dessins

Ces objets, caractéristiques et avantages, ainsi que d'autres seront exposés en détail dans la description suivante d'exemples de réalisation particuliers faite à titre non limitatif en relation avec les figures jointes parmi lesquelles :

la figure 1 représente, de façon partielle et schématique, un exemple de réalisation d'un dispositif de contrôle d'au moins un paramètre d'ouvrabilité d'un béton dans le réservoir d'un malaxeur à axe de rotation non-vertical selon un mode de réalisation de l'invention ;
la figure 2 représente, sous la forme d'un schéma par blocs, un exemple de réalisation selon l'invention d'un procédé de contrôle d'un paramètre d'ouvrabilité d'un béton ;
la figure 3 représente un exemple d'évolution du couple entraînant en rotation le réservoir d'un malaxeur à axe de rotation non-vertical en fonction de la vitesse de rotation du réservoir pour deux bétons de formulations différentes ;
la figure 4 représente un exemple d'évolution de la contrainte de cisaillement $\tau$ en fonction du gradient de vitesse $\dot{\gamma}$ pour ces deux bétons, mesurée par un rhéomètre ;
la figure 5 représente des courbes d'évolution de la contrainte de cisaillement $\tau$ en fonction du gradient de vitesse $\dot{\gamma}$ de bétons de formulations différentes, mesurées par un rhéomètre ;

la figure 6 représente un exemple de courbe d'évolution de la relation de passage G ;

la figure 7 représente un exemple de courbe d'évolution de la relation de passage T ;

la figure 8 représente, sous la forme d'un schéma par blocs, un exemple de réalisation selon l'invention plus détaillé d'un procédé de contrôle d'un paramètre d'ouvrabilité d'un béton ;

la figure 9 représente un exemple d'évolution de la pression hydraulique mesurée en entrée du moteur hydraulique ou du différentiel de pression entre l'entrée et la sortie du moteur hydraulique entraînant en rotation le réservoir du malaxeur ; et

la figure 10 représente, sous la forme d'un schéma par blocs, un exemple de réalisation d'un procédé d'ajustement de l'affaissement d'un béton selon l'invention.

Par souci de clarté, des mêmes éléments ont été désignés par des mêmes références dans les différentes figures. En outre, seuls les éléments nécessaires à la compréhension de l'invention sont représentés sur les figures et sont décrits.

## Description détaillée

Dans la suite de la description, on emploie indifféremment les expressions viscosité, viscosité apparente et viscosité dynamique pour désigner le rapport entre la contrainte de cisaillement et le gradient de vitesse du béton.

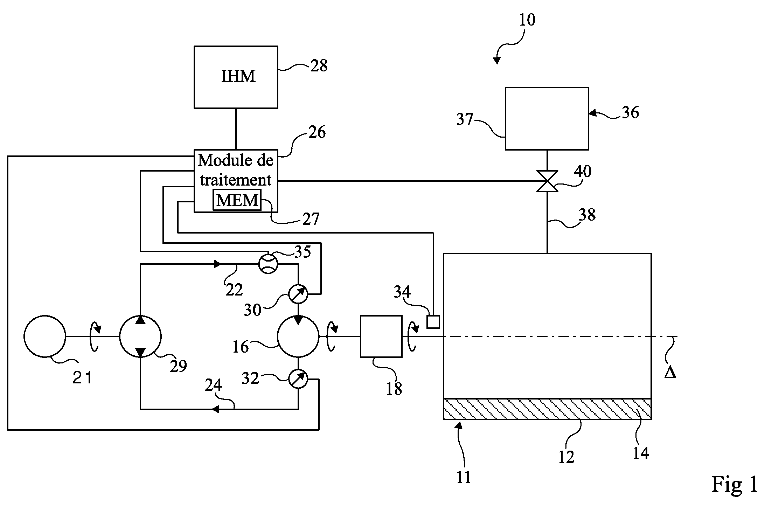

La figure 1 représente un exemple de réalisation d'un dispositif 10 de contrôle d'au moins un paramètre d'ouvrabilité d'un béton selon un exemple de réalisation de l'invention.

Un béton est un mélange de granulats agglomérés par un liant et de l'eau.

Le liant hydraulique est un matériau qui prend et durcit par hydratation. De préférence, le liant hydraulique est un ciment, notamment un ciment Portland, par exemple un ciment de type CEM I, CEM II, CEM III, CEM IV ou CEM V selon la norme européenne NF EN 197-1 de février 2001.

Le béton peut être un mélange d'un liant hydraulique, de granulats, d'eau, éventuellement d'adjuvants, et éventuellement d'additions minérales. Il s'agit, par exemple, d'un béton hautes performances, d'un béton très hautes performances, d'un béton autoplaçant, d'un béton autonivelant, d'un béton autocompactant, d'un béton fibré, d'un béton prêt à l'emploi ou d'un béton coloré. Le terme béton comprend les mortiers. Dans ce cas, le béton comprend un mélange de liant hydraulique, de sable, d'eau et éventuellement d'additifs et éventuellement d'additions minérales.

Les additions minérales sont généralement, par exemple, des matériaux pouzzolaniques (par exemple tels que définis dans la norme européenne NF EN 197-1 de février 2001 paragraphe 5.2.3), des fumées de silice (par exemple telles que définies dans la norme européenne NF EN 197-1 de février 2001 paragraphe 5.2.7 ou telles que définies dans la norme « Béton » prEN 13263 :1998 ou NF P 18-502), des laitiers (par exemple tels que définis dans la norme européenne NF EN 197-1 paragraphe 5.2.2 ou tels que définis dans la norme « Béton » NF P 18-506), des schistes calcinés (par exemple tels que définis dans la norme européenne NF EN 197-1 de février 2001 paragraphe 5.2.5), des matériaux contenant du carbonate de calcium, par exemple du calcaire (par exemple tels que définis dans la norme européenne NF EN 197-1 de février 2001 paragraphe 5.2.6 ou tels que définis dans la norme « Béton » NF P 18-508), des additions siliceuses (par exemple telles que définies dans la norme « Béton » NF P 18-509), les métakaolins ou leurs mélanges.

Le liant peut être un liant hydrocarboné, c'est-à-dire une substance composée d'un mélange d'hydrocarbures, très visqueuse voire solide à la température ambiante. Le liant hydrocarboné peut, par exemple, être du bitume naturel ou du bitume brut dérivé du pétrole.

Le béton peut être un mélange d'un liant hydrocarboné et de granulats, comme par exemple du béton bitumineux, des graves-bitumes, de l'asphalte, ou des enduits superficiels à base d'émulsion de bitume. Un béton à liant hydrocarboné selon l'invention peut en outre comprendre des additifs usuels, comme par exemple des dopes d'adhésivité ou des fibres (en verre, en cellulose ou en amiante, par exemple). Un béton à liant hydrocarboné peut, en outre, comprendre des matériaux recyclés, comme par exemple des bardeaux de toiture, du verre ou du béton de ciment.

Les granulats comprennent des graviers, des gravillons et/ou du sable. Le sable correspond à un granulat ayant une granulométrie strictement inférieure à 4 mm. Les gravillons correspondent à des granulats ayant une granulométrie comprise de 4 à 20 mm. Les graviers correspondent à des granulats ayant une granulométrie strictement supérieure à 20 mm.

Les exemples de réalisation de l'invention sont décrits par la suite pour un béton comprenant un liant hydraulique.

Le dispositif 10 comprend un malaxeur 11 comportant un réservoir 12 dans lequel est disposé un béton 14. A titre d'exemple, le malaxeur 11 correspond à un camion-toupie utilisé pour le transport du béton depuis un site de fabrication du béton jusqu'à un site d'utilisation du béton. A titre de variante, le malaxeur 11 peut être un malaxeur fixe à axe de rotation non-vertical utilisé pour la fabrication du béton. De préférence, l'axe du malaxeur est incliné par rapport à la direction horizontale d'un angle inférieur ou égal à 45°.

Le malaxeur 11 comprend un moteur hydraulique 16 qui entraîne en rotation le réservoir 12 autour d'un axe Δ non-vertical par l'intermédiaire d'un réducteur 18. Dans le cas d'un réservoir 12 d'un camion-toupie, l'axe Δ peut être légèrement incliné par rapport à la direction horizontale. A titre d'exemple, le volume V de béton 14 dans le réservoir 12 peut varier de 0,5 m$^3$ à 8 m$^3$, dans certains cas, jusqu'à 15 m$^3$.

La vitesse de rotation du réservoir 12 autour de l'axe Δ peut être exprimée en radians par seconde et est alors notée ω dans la suite de la description ou être exprimée en tours par minute et est alors notée N dans la suite de la description. A titre d'exemple, dans le cas d'un camion-toupie, la vitesse de rotation N peut varier de 1 tr/min à 20 tr/min par minute. A titre d'exemple, pour le transport du béton, la vitesse de rotation du réservoir 12 varie généralement de 1 tr/min à 6 tr/min. Pour une opération de malaxage du béton lors de la fabrication du béton ou avant l'utilisation du béton sur le site d'utilisation du béton, la vitesse de rotation du réservoir 12 est généralement supérieure à 6 tr/min, et peut atteindre 15 tr/min.

L'actionnement du moteur hydraulique 16 peut être réalisé par la mise en circulation d'un fluide hydraulique par une pompe hydraulique 20 reliée au moteur hydraulique 16 par une conduite 22 d'apport du fluide hydraulique de la pompe hydraulique 20 au moteur hydraulique 16 et par une conduite 24 de retour du fluide hydraulique du moteur hydraulique 16 à la pompe hydraulique 20. La pompe hydraulique 20 peut être entraînée en rotation par un moteur 21 , par exemple le moteur du camion toupie.

Le dispositif 10 comprend un module de traitement 26, comprenant, par exemple, un microcontrôleur, comportant une mémoire (MEM) 27. Le module de traitement 26 est relié à une interface homme/machine 28 (IHM) comprenant, par exemple, un écran d'affichage, un écran tactile, un clavier, etc.

Le dispositif 10 comprend un premier capteur 30 de pression hydraulique adapté à mesurer la pression du fluide hydraulique en amont du moteur hydraulique 16. Le dispositif 10 comprend un second capteur 32 de pression hydraulique adapté à mesurer la pression du fluide hydraulique en aval du moteur hydraulique 16. Les capteurs 30 et 32 sont reliés au module de traitement 26. Une alternative peut être d'utiliser un capteur de pression différentielle relié à l'entrée et à la sortie du moteur hydraulique 16.

Le dispositif 10 peut comprendre, en outre, un capteur de vitesse 34, relié au module de traitement 26, mesurant la vitesse de rotation du réservoir 12. Il peut s'agir d'un capteur de vitesse de rotation passif, notamment de type inductif, ou d'un capteur de vitesse de rotation actif, notamment de type magnétorésistif ou à effet Hall. Le dispositif 10 peut comprendre un capteur 35 adapté à mesurer le débit du fluide hydraulique circulant dans les conduites 22 et/ou 24, de préférence dans la conduite 22 en entrée du moteur hydraulique 16.

Le dispositif 10 comprend un système 36 d'ajout d'eau, d'un adjuvant ou d'un mélange d'adjuvants dans le béton 14. L'adjuvant ou le mélange d'adjuvants peut être ajouté à l'eau. Le système 36 peut comprendre une cuve 37 contenant l'eau, l'adjuvant ou le mélange d'adjudants. La cuve 37 est reliée au réservoir 12 par une conduite 38 munie d'une vanne 40. La vanne 40 peut être commandée par le module de traitement 26. A titre d'exemple, il peut s'agir d'une vanne à air comprimé, l'actionnement de la vanne 40 étant obtenu par mise en circulation d'air comprimé sous la commande du module de traitement 26. A titre de variante, le système 36 peut comprendre une pompe, non représentée, reliée à la cuve 37.

Les adjuvants peuvent correspondre à des adjuvants ajoutés de façon habituelle dans les bétons, notamment un réducteur d'eau/plastifiant, un superplastifiant, un agent retardant, un accélérateur de prise, un agent épaississant ou un agent de modification de viscosité.

Avantageusement, le dispositif 10 permet de déterminer la composition finale du béton, juste avant son utilisation sur site, avec les différents ajouts (notamment l'eau, l'adjuvant ou le mélange d'adjuvants) et éventuellement l'édition de cette composition mise à jour à réception du béton par le client sur le site d'utilisation du béton.

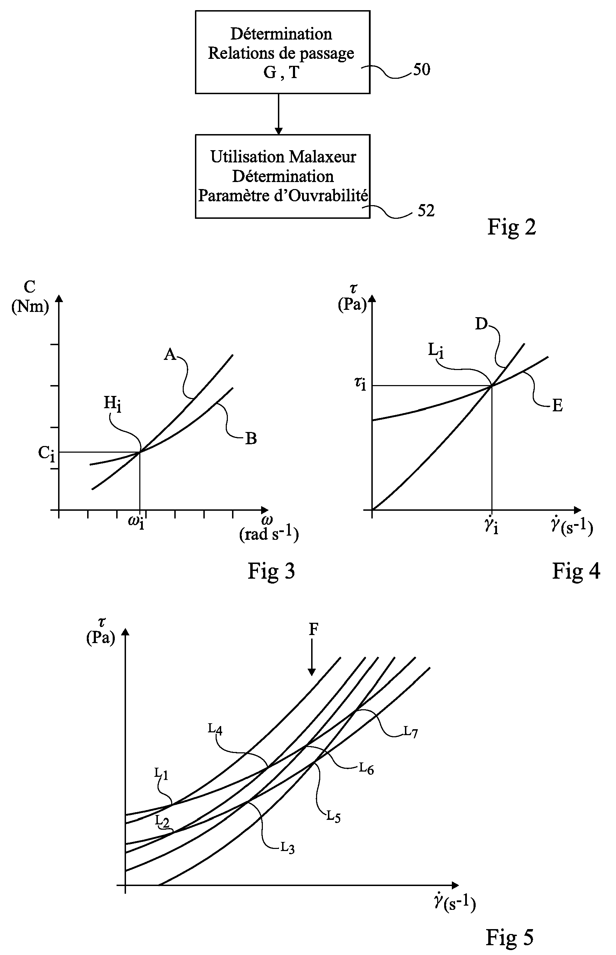

La figure 2 représente, sous la forme d'un schéma par blocs, un exemple de réalisation selon l'invention d'un procédé de contrôle d'au moins un paramètre d'ouvrabilité d'un béton. Le procédé comprend deux étapes 50 et 52. L'étape 50 est à réaliser une seule fois avant l'utilisation prévue du malaxeur 11. L'étape 52 peut être mise en oeuvre à chaque utilisation du malaxeur 11. L'étape 52 peut être répétée plusieurs fois au cours de l'utilisation du malaxeur 11.

L'étape 50 comprend la détermination de relations de passage G et T et l'étape 52 comprend la détermination (et éventuellement l'ajustement) d'un paramètre d'ouvrabilité à partir des relations de passage G et T.

Le paramètre d'ouvrabilité peut correspondre à l'affaissement, à l'étalement, à la contrainte seuil, au temps d'écoulement ou à la viscosité d'un béton.

La contrainte seuil d'un béton est la contrainte au-delà de laquelle le béton commence à s'écouler. Lorsque la contrainte de cisaillement $\tau$ est exprimée en fonction du gradient de vitesse $\dot{\gamma}$ (ou taux de cisaillement), la contrainte seuil $\tau_0$ correspond à la contrainte de cisaillement pour un gradient de vitesse extrapolé à zéro. La viscosité apparente $\eta$ d'un béton correspond au rapport entre la contrainte de cisaillement $\tau$ et le gradient de vitesse $\dot{\gamma}$. Elle n'est pas toujours constante pour un béton mais, dans certains cas, elle peut être constante.

De façon générale, le béton présent dans le réservoir 12 peut être considéré comme un fluide de Herschel-Bulkley. L'expression de la contrainte de cisaillement $\tau$ en fonction du gradient de vitesse $\dot{\gamma}$ est donnée par la relation (1) suivante :

$$\tau = \tau_0 + k . \dot{\gamma}^p \tag{1}$$

où k et p sont des nombres réels positifs. Pour certains types de béton, notamment les bétons ordinaires, le béton peut être considéré comme un fluide de Bingham. L'expression (1) se simplifie alors de la façon suivante :

$$\tau = \tau_0 + \eta_p . \dot{\gamma} \tag{2}$$

où $\eta_p$ est la viscosité plastique du béton.

La relation de passage G est une fonction qui permet d'obtenir le gradient de vitesse $\dot{\gamma}$ à partir de la vitesse de rotation $\omega$ du réservoir 12 selon la relation (3) suivante :

$$\dot{\gamma} = G(\omega) . \omega \tag{3}$$

La relation de passage T est une fonction qui permet de déterminer la contrainte de cisaillement $\tau$ à partir du couple C d'entraînement en rotation du réservoir 12 selon la relation (4) suivante :

$$\tau = T(\omega) . C \tag{4}$$

Les relations de passage G et T sont des fonctions qui ne sont pas constantes et peuvent dépendre notamment de la vitesse de rotation $\omega$. De préférence, les relations de passage G et T dépendent seulement de la vitesse de rotation $\omega$.

A titre d'exemple, les relations de passage G et T peuvent être exprimées sous la forme de polynômes selon les relations (5) et (6) suivantes :

$$G = \sum_{j=0}^{M} G_j \omega^j \tag{5}$$

$$T = \sum_{j=0}^{M} T_j \omega^j \tag{6}$$

où $G_j$ et $T_j$ sont des nombres réels et M est un nombre entier supérieur ou égal à 1.

Les relations de passage G et T dépendent des caractéristiques du malaxeur 11 mais sont indépendantes des formulations des bétons susceptibles d'être disposés dans le réservoir 12 du malaxeur 11.

Le procédé de détermination des relations de passage G et T se base sur le principe suivant : deux bétons pour lesquels il est mesuré la même contrainte de cisaillement $\tau_i$ pour un gradient de cisaillement $\dot{\gamma}_i$ donné développent, dans le malaxeur 11, le même couple d'entraînement $C_i$ du réservoir 12 du malaxeur 11 pour une vitesse de rotation $\omega_i$ donnée du réservoir 12.

La figure 3 représente les courbes d'évolution A et B du couple d'entraînement C du réservoir 12 en fonction de la vitesse de rotation $\omega$ du réservoir 12 pour deux bétons de formulations différentes et la figure 4 représente les courbes d'évolution D et E de la contrainte de cisaillement $\tau$ en fonction du gradient de vitesse $\dot{\gamma}$ pour ces deux bétons. Les courbes A et B sont déterminées en utilisant le malaxeur 11. Les courbes D et E sont déterminées en utilisant un rhéomètre.

Les courbes A et B se coupent en un point $H_i$. Les courbes D et E se coupent en un point $L_i$. Au point $H_i$, les deux bétons ont, dans le réservoir 12, le même couple $C_i$ à la vitesse de rotation $\omega_i$. Au point $L_i$, les deux bétons ont la même contrainte de cisaillement $\tau_i$ au gradient de vitesse $\dot{\gamma}_i$. Les deux bétons sont donc dans le même état rhéologique au point $L_i$ et au point $H_i$, c'est-à-dire qu'ils développent la même contrainte $\tau_i$ pour le gradient de vitesse $\dot{\gamma}_i$.

Selon un exemple de réalisation selon l'invention, le procédé de détermination des expressions des relations de passage G et T en fonction de la vitesse de rotation $\omega$ consiste à déterminer les courbes d'évolution du couple d'entraînement C en fonction de la vitesse de rotation $\omega$ et les courbes d'évolution de la contrainte de cisaillement $\tau$ en fonction du gradient de vitesse $\dot{\gamma}$ pour plusieurs bétons de façon à obtenir plusieurs points de croisement $H_i$ et $L_i$.

La figure 5 représente, à titre d'exemple, plusieurs courbes F d'évolution de la contrainte de cisaillement $\tau$ en fonction du gradient de vitesse $\dot{\gamma}$ pour six bétons de formulations différentes. Ces courbes se coupent en des points d'intersection $L_1$ à $L_7$.

Selon un premier exemple de procédé de détermination des expressions des relations de passage G et T, pour chaque point d'intersection $H_i$ entre deux courbes d'évolution du couple d'entraînement C en fonction de la vitesse de rotation $\omega$ d'une paire de bétons et pour le point d'intersection $L_i$ entre les courbes d'évolution de la contrainte de cisaillement $\tau$ en fonction du gradient de vitesse $\dot{\gamma}$ de la même paire de bétons, il est déterminé la valeur $G_i^{CC}$ de la relation de passage G et la valeur $T_i^{CC}$ de la relation de passage T selon les relations (7) et (8) suivantes :

$$G_i^{CC} = \frac{\gamma_i}{\omega_i} \tag{7}$$

$$T_i^{CC} = \frac{\tau_i}{C_i} \tag{8}$$

La détermination du couple d'entraînement est explicitée plus en détail par la suite.

Les relations de passages G et T peuvent être recherchées, à titre d'exemple, sous la forme des expressions (5) et (6) décrites précédemment en déterminant les paramètres $G_j$ et $T_j$ pour lesquels les courbes des relations de passage G et T passent par les valeurs $G_i^{CC}$ et $T_i^{CC}$ ou s'approchent au mieux de ces valeurs selon des méthodes d'interpolation ou d'approximation. Une fois déterminées, les relations de passage G et T sont stockées dans la mémoire 27 du module de traitement 26.

Selon un deuxième exemple de procédé de détermination des relations de passage G et T, les relations de passage G et T sont déterminées à partir de valeurs $G_i^{Alt}$ et $T_i^{Alt}$ aux points de croisement d'indice i. La valeur $G_i^{Alt}$ de la relation de passage G et la valeur $T_i^{Alt}$ de la relation T aux points de croisement d'indice i sont obtenues par les relations (9) et (10) suivantes :

$$G_i^{Alt} = \sqrt{\frac{C_i}{V \cdot \eta_i \cdot \omega_i}} \tag{9}$$

$$T_i^{Alt} = \frac{1}{G_i^{Alt} \cdot V} \tag{10}$$

où V est le volume du béton 14 dans le réservoir 12 et $\eta_i$ est la viscosité apparente du béton au point de croisement $L_i$.

Les relations de passages G et T peuvent alors être recherchées, à titre d'exemple, sous la forme des expressions (5) et (6) décrites précédemment en déterminant les paramètres $G_j$ et $T_j$ pour lesquels les courbes des relations de passage G et T passent par les valeurs $G_i^{Alt}$ et $T_i^{Alt}$ ou s'approchent au mieux de ces valeurs selon des méthodes d'interpolation ou d'approximation. Le deuxième exemple de procédé de détermination des relations de passage T et G présente l'avantage d'être moins sensible aux incertitudes de mesure que le premier exemple.

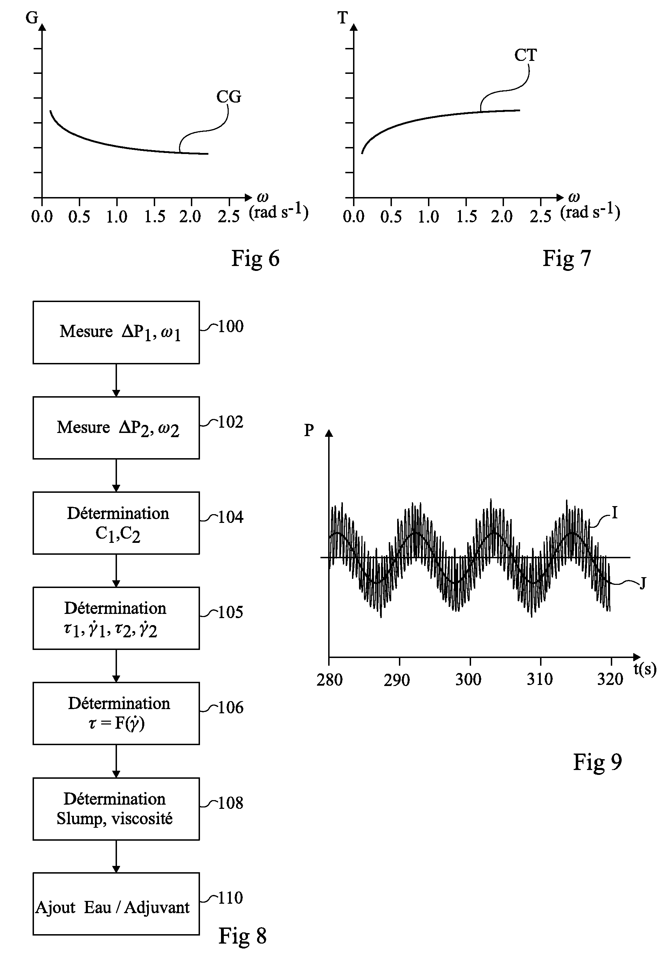

Les figures 6 et 7 représentent deux exemples de courbes d'évolution CG et CT respectivement des relations de passage G et T.

La figure 8 représente, sous la forme d'un schéma par blocs, un exemple de réalisation plus détaillé selon l'invention de l'étape 52 du procédé illustré en figure 2.

A l'étape 100, le malaxeur 11 est commandé à un premier régime de fonctionnement. Le module de traitement 26 détermine une première valeur $\Delta P_1$ de la différence de pression $\Delta P$ du fluide hydraulique entre l'amont et l'aval du moteur hydraulique 16 et une première valeur $\omega_1$ de la vitesse de rotation $\omega$ du réservoir 12. La différence de pression $\Delta P$ du fluide hydraulique entre l'amont et l'aval du moteur hydraulique 16 peut être mesurée par les capteurs de pression 30 et 32. La vitesse de rotation $\omega$ du réservoir 12 peut être déterminée directement par le capteur 34 ou indirectement à partir de la mesure du débit du liquide hydraulique traversant le moteur hydraulique 16. Le procédé se poursuit à l'étape 102.

A l'étape 102, le malaxeur 11 est commandé à un deuxième régime de fonctionnement, différent du premier régime de fonctionnement. Ceci signifie que la vitesse de rotation du réservoir 12 au premier régime de fonctionnement est différente de la vitesse de rotation du réservoir 12 au deuxième régime de fonctionnement. Le module de traitement 26 détermine alors une deuxième valeur $\Delta P_2$ de la différence de pression $\Delta P$ du fluide hydraulique entre l'amont et l'aval du moteur hydraulique 16 et une deuxième valeur $\omega_2$ de la vitesse de rotation $\omega$. Les étapes 100 et 102 peuvent être répétées à plusieurs reprises pour d'autres régimes de fonctionnement du malaxeur 11. De préférence, le malaxeur 11 peut, en outre, être commandé à un troisième régime de fonctionnement, différent des premier et deuxième régimes de fonctionnement. Le module de traitement 26 détermine alors une troisième valeur $\Delta P_3$ de la différence de pression $\Delta P$ du fluide hydraulique entre l'amont et l'aval du moteur hydraulique 16 et une troisième valeur $\omega_3$ de la vitesse de rotation $\omega$. Le procédé se poursuit ensuite à l'étape 104.

Les étapes 100 et 102 peuvent être mises en oeuvre de façon automatique ou sous une action volontaire du conducteur du camion-toupie. Elles peuvent être mises en oeuvre lors du transport du béton et/ou de préférence lorsque le camion-toupie est à l'arrêt.

A l'étape 104, le module de traitement 26 détermine des valeurs $C_1$ et $C_2$ du couple $C$ entraînant le réservoir 12 respectivement à partir des valeurs $\Delta P_1$ et $\Delta P_2$ de la différence de pression $\Delta P$ comme cela sera décrit plus en détail par la suite. Le procédé se poursuit à l'étape 105.

A l'étape 105, le module de traitement 26 détermine une première valeur $\tau_1$ de la contrainte de cisaillement $\tau$ et une première valeur $\dot{\gamma}_1$ du gradient de vitesse (ou taux de cisaillement) $\dot{\gamma}$ du béton au premier régime de fonctionnement à partir des valeurs $\Delta P_1$ et $\omega_1$ selon les relations (11) et (12) suivantes :

$$\dot{\gamma}_1 = G(\omega_1) \cdot \omega_1 \tag{11}$$

$$\tau_1 = T(\omega_1) \cdot C_1 \tag{12}$$

où $G(\omega_1)$ est la valeur de la relation de passage $G$ à la vitesse de rotation $\omega_1$ et $T(\omega_1)$ est la valeur de la relation de passage $T$ à la vitesse de rotation $\omega_1$.

Le module de traitement 26 détermine, en outre, une deuxième valeur $\tau_2$ de la contrainte de cisaillement $\tau$ et une deuxième valeur $\dot{\gamma}_2$ du gradient de vitesse (ou taux de cisaillement) $\dot{\gamma}$ du béton au deuxième régime de fonctionnement à partir des valeurs $\Delta P_2$ et $\omega_2$ selon les relations (13) et (14) suivantes :

$$\dot{\gamma}_2 = G(\omega_2) \cdot \omega_2 \tag{13}$$

$$\tau_2 = T(\omega_2) \cdot C_2 \tag{14}$$

où $G(\omega_2)$ est la valeur de la relation de passage $G$ à la vitesse de rotation $\omega_2$ et $T(\omega_2)$ est la valeur de la relation de passage $T$ à la vitesse de rotation $\omega_2$.

De préférence, le module de traitement 26 peut, en outre, déterminer une troisième valeur $\tau_3$ de la contrainte de cisaillement $\tau$ et une troisième valeur $\dot{\gamma}_3$ du gradient de vitesse (ou taux de cisaillement) $\dot{\gamma}$ du béton au troisième régime de fonctionnement à partir des valeurs $\Delta P_3$ et $\omega_3$ selon les relations (15) et (16) suivantes :

$$\dot{\gamma}_3 = G(\omega_3) \cdot \omega_3 \tag{15}$$

$$\tau_3 = T(\omega_3) \cdot C_3 \tag{16}$$

où $G(\omega_3)$ est la valeur de la relation de passage $G$ à la vitesse de rotation $\omega_3$ et $T(\omega_3)$ est la valeur de la relation de passage $T$ à la vitesse de rotation $\omega_3$.

Selon une variante, le module de traitement 26 peut, en outre, déterminer d'autres valeurs supplémentaires de la contrainte de cisaillement $\tau$ et du gradient de cisaillement $\dot{\gamma}$, en plus des premières, deuxièmes et troisièmes valeurs mentionnées ci-dessus.

Le procédé se poursuit à l'étape 106.

A l'étape 106, le module de traitement 26 détermine l'expression de la contrainte de cisaillement $\tau$ en fonction du gradient de vitesse $\dot{\gamma}$ à partir des couples de valeurs $(\tau_1, \dot{\gamma}_1)$ et $(\tau_2, \dot{\gamma}2)$ (et, de préférence, en outre, le couple de

valeurs $(\tau_3, \dot{\gamma}_3)$). A l'étape 106, le module de traitement 26 peut rechercher l'expression de $\tau$ sous la forme des expressions (1) ou (2) en déterminant les paramètres $\tau_0$, k et p (ou $\eta_p$) pour lesquels la courbe d'évolution de la contrainte de cisaillement $\tau$ en fonction du gradient de vitesse $\dot{\gamma}$ passe par les points $(\tau_1, \dot{\gamma}_1)$ et $(\tau_2, \dot{\gamma}_2)$ (et, de préférence, en outre le point $(\tau_3, \dot{\gamma}_3)$) ou s'approche au mieux de ces valeurs selon des méthodes d'interpolation ou d'approximation. Le procédé se poursuit à l'étape 108.

A l'étape 108, le module de traitement 26 détermine le paramètre d'ouvrabilité ou les paramètres d'ouvrabilité souhaités à partir de l'expression précédente. La contrainte seuil $\tau_0$ peut être déterminée directement à partir de la relation (1) ou (2). L'affaissement ou l'étalement du béton peut être déterminé à partir de la contrainte seuil $\tau_0$. A titre d'exemple, l'affaissement (slump) ou l'étalement (slump flow) peut être obtenu selon les relations (17) et (18) suivantes :

$$\tau_0 = E_0 + E_1 \cdot \text{Slump}^{\alpha} \qquad (17)$$

$$\frac{\tau_0}{\rho} = E_2 + E_3 \cdot \text{Slump}^{\alpha} \qquad (18)$$

où $E_0$, $E_1$, $E_2$, $E_3$, et $\alpha$ sont des nombres réels déterminés au préalable et qui sont indépendants du malaxeur 11 et de la formulation du béton et où $\rho$ est la masse volumique du béton. La viscosité apparente $\eta$ du béton correspond au rapport entre la contrainte de cisaillement $\tau$ et le gradient de vitesse $\dot{\gamma}$. Le module de traitement 26 peut, en outre, commander l'interface 28 pour afficher le ou les paramètres d'ouvrabilité mesurés. En outre, le ou les paramètres d'ouvrabilité mesurés et l'instant de mesure peuvent être mémorisés. Le procédé se poursuit à l'étape 110.

A l'étape 110, le module de traitement 26 peut commander l'ajout dans le béton d'eau ou d'adjuvants pour modifier le ou les paramètres d'ouvrabilité mesurés. L'étape 110 peut ne pas être présente.

Dans l'exemple de réalisation de procédé selon l'invention décrit en relation avec la figure 8, aux étapes 100 et 102, les pressions sont déterminées à partir des capteurs de pression 30 et 32.

La figure 9 représente un exemple de courbe I d'évolution du signal fourni par le capteur 30 pour plusieurs rotations du réservoir 12. La courbe J représente l'évolution du signal fourni par le capteur 30 après une opération de filtrage passe-bas. La courbe J peut comprendre des oscillations au cours d'une révolution du réservoir 12 qui peuvent notamment être dues à des défauts d'équilibrage du réservoir 12, à la nature du béton, etc. La fréquence des oscillations correspond sensiblement à la fréquence de rotation du réservoir 12. Aux étapes 100 et 102 décrites précédemment, la pression mesurée correspond à une pression moyenne. Il est avantageux, pour déterminer la pression moyenne, de considérer au minimum une révolution complète du réservoir 12. C'est pourquoi le nombre Trame d'échantillons successifs utilisés pour déterminer la pression moyenne varie en fonction de la vitesse de rotation $\omega$ du réservoir 12. Le nombre Trame d'échantillons dépend du nombre d'oscillations $Nb_{osci}$ de la courbe pendant une révolution du réservoir 12, de la vitesse de rotation N du réservoir 12 et de la fréquence f d'acquisition des échantillons de pression selon la relation suivante (19) :

$$\text{Trame} = 60 \, \frac{Nb_{osc} \cdot f}{N} \qquad (19)$$

Les échantillons sont considérés comme stables lorsque, pour chaque échantillon mesuré parmi le nombre Trame d'échantillons, la vitesse de rotation N du réservoir 12 varie peu par rapport à une vitesse de rotation moyenne pour le nombre Trame d'échantillons, par exemple varie de moins de 1 tour par minute par rapport à la vitesse de rotation moyenne pour le nombre Trame d'échantillons. La pression moyenne est mesurée seulement lorsque les échantillons sont stables.

Le signal fourni par le capteur 30 est noté $P_e$ et le signal en sortie du moteur hydraulique 16 obtenu à partir du capteur 32 est noté $P_s$. La pression différentielle $\Delta P$ est égale à la différence entre les pressions d'entrée $P_e$ et de sortie $P_s$. La valeur moyenne de la pression différentielle est obtenue en faisant la moyenne des valeurs de la pression différentielle $\Delta P$ de l'ensemble des échantillons du nombre Trame d'échantillons.

La relation entre la pression différentielle $\Delta P$ et le couple d'entraînement C est obtenue de la façon suivante. La puissance mécanique $P_M$ utile à la rotation de la toupie est donnée par la relation (20) suivante :

$$P_M = C \cdot \omega \qquad (20)$$

Lorsque le moteur hydraulique 16 fonctionne dans une plage de fonctionnement linéaire, la puissance hydraulique $P_{hy}$ du moteur hydraulique 16 est donnée par la relation (21) suivante :

$$P_{hy} = \Delta P \cdot Q \qquad\qquad (21)$$

où Q est le débit du fluide hydraulique, exprimé en $m^3/s$, entraînant le moteur hydraulique 16. Le débit Q est donné par la relation (22) suivante :

$$Q = C_y \cdot n_m \qquad\qquad (22)$$

où $n_m$ est la vitesse de rotation du moteur hydraulique 16 exprimée en tours par seconde et Cy est la cylindrée du moteur hydraulique 16. La cylindrée $C_y$, exprimée en $m^3/tr$, correspond au volume de fluide hydraulique qui transite dans le moteur hydraulique 16 pendant une révolution du moteur hydraulique 16.

En considérant que la puissance mécanique $P_M$ est égale au produit de la puissance hydraulique $P_{hy}$ et d'un facteur de rendement R et que la vitesse de rotation $n_m$ du moteur hydraulique 16 est égale au produit de la vitesse de rotation $\omega$ du réservoir 12 et d'un facteur de réduction $K_r$, on obtient la relation (23) suivante :

$$C = R \cdot \Delta P \cdot C_y \cdot K_r \qquad\qquad (23)$$

Le couple d'entraînement C peut être déterminé en remplaçant dans l'expression (23) la différence de pression $\Delta P$ par la pression d'entrée $P_e$. Toutefois, les inventeurs ont mis en évidence que la précision de la détermination du couple d'entraînement C est augmentée en utilisant la différence de pression $\Delta P$ plutôt que seulement la pression d'entrée $P_e$.

Le couple d'entraînement C que l'on cherche à mesurer doit être le plus fidèlement possible représentatif du comportement du béton et non d'autres paramètres comme par exemple les frottements entre le réservoir 12 et le système de support du réservoir 12 ou la masse du réservoir 12 à vide.

Il peut donc être avantageux de mesurer la courbe d'évolution de la pression d'entrée $P_{e0}$ et la courbe d'évolution de la pression de sortie $P_{s0}$ en fonction de la vitesse de rotation $\omega$ du réservoir 12 en l'absence de béton dans le réservoir 12 et de retrancher la valeur $P_{e0}$ à la vitesse de rotation de la mesure de la pression $P_e$ mesurée et la valeur $P_{s0}$ à la vitesse de rotation de la mesure de la pression $P_s$ mesurée lors de la détermination de $\Delta P$.

En appelant $\Delta P_0$ la différence de pression à vide, c'est-à-dire la différence entre $P_{e0}$ et $P_{s0}$, la relation (24) suivante peut alors être utilisée à la place de la relation (23) précédente :

$$C = R \cdot (\Delta P - \Delta P_0) \cdot C_y \cdot K_r \qquad\qquad (24)$$

Les inventeurs ont, en outre, mis en évidence que la précision de la détermination du couple d'entraînement C est augmentée en utilisant la pression différentielle corrigée $\Delta P - \Delta P_0$ plutôt que la pression différentielle $\Delta P$ seule.

La vitesse de rotation $\omega$ du réservoir 12 peut être déterminée directement à partir du capteur de vitesse de rotation 34 ou peut être déterminée de façon indirecte à partir du débit d'huile Q mesuré par le capteur 35 selon la relation (25) suivante :

$$\omega = \frac{Q}{K_r \cdot C_y} \qquad\qquad (25)$$

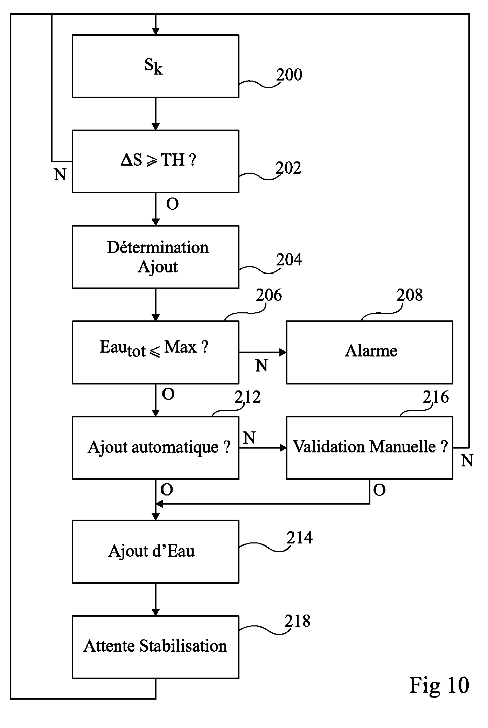

La figure 10 représente, sous la forme d'un schéma par blocs, un exemple de réalisation de l'étape 110 du procédé illustré en figure 8 dans le cas où, à l'étape 108, le procédé fournit une valeur d'affaissement et dans le cas où l'affaissement est ajusté par l'ajout d'eau au béton. Ce procédé d'ajustement peut également être employé pour le contrôle de l'étalement, du temps d'écoulement, de la viscosité, ou de la contrainte seuil.

A l'étape 200, le module de traitement 26 détermine la dernière valeur $S_k$ de l'affaissement. La dernière valeur d'affaissement $S_k$ peut correspondre, par exemple, à la moyenne de dernières valeurs d'affaissement, par exemple les 5 dernières valeurs d'affaissement, obtenues à l'étape 108. Le procédé se poursuit à l'étape 202.

A l'étape 202, le module de traitement 26 détermine la différence $\Delta S$ entre une valeur d'affaissement de comparaison Se et la dernière valeur d'affaissement $S_k$. Si la différence $\Delta S$ est supérieure à un seuil TH, le procédé se poursuit à l'étape 204. Si la différence $\Delta S$ est inférieure au seuil TH, le procédé retourne à l'étape 200. Le seuil TH traduit la variation d'affaissement qui est acceptable. De façon typique pour un béton ordinaire, le seuil TH peut être de l'ordre de 30 mm.

A l'étape 204, le module de traitement 26 détermine la quantité d'eau (Ajout) à ajouter. La quantité Ajout peut être déterminée par la relation (26) suivante :

$$Ajout = Tx_{eau} \cdot V \cdot \Delta S \cdot K_S \qquad (26)$$

où $Tx_{eau}$ correspond à la quantité d'eau à ajouter par mètre cube de béton et par millimètre de variation d'affaissement, $K_s$ est un coefficient de sécurité et V est le volume de béton. La quantité d'eau $Tx_{eau}$ est, par exemple, comprise entre 0,1 $L/m^3/mm$ et 3 $L/m^3/mm$ et le coefficient de sécurité $K_s$ est par exemple compris entre 0 et 1. Le procédé se poursuit à l'étape 206.

A l'étape 206, le module de traitement 26 détermine la quantité totale d'eau ajoutée ($Eau_{tot}$) au béton depuis la mise en place du béton dans le réservoir 12. La quantité totale d'eau ajoutée $Eau_{tot}$ correspond à la somme des ajouts successifs déjà réalisés depuis la mise en place du béton dans le réservoir 12, de l'ajout d'eau (Ajout) calculé à l'étape précédente et non encore réalisé, et de la quantité d'eau initialement introduite dans le béton avant chargement dans le réservoir. La quantité totale d'eau ($Eau_{tot}$) est comparée à une quantité maximale d'eau (Max) pouvant entrer dans la composition dudit béton. Si la quantité d'eau $Eau_{tot}$ est strictement supérieure à Max, le procédé se poursuit à l'étape 208. Si la quantité d'eau $Eau_{tot}$ est inférieure ou égale à Max, le procédé se poursuit à l'étape 212.

A l'étape 208, le module de traitement 26 envoie une alarme, par exemple au conducteur du camion-toupie, par l'intermédiaire de l'interface 28.

A l'étape 212, le module de traitement 26 détermine si l'ajout de la quantité d'eau (Ajout) doit être réalisé automatiquement. Si la quantité d'eau (Ajout) doit être ajoutée de façon automatique, le procédé se poursuit à l'étape 214. Si la quantité d'eau (Ajout) ne doit pas être ajoutée de façon automatique, le procédé se poursuit à l'étape 216.

A l'étape 216, le module de traitement 26 attend qu'une validation manuelle soit réalisée, par exemple, par le conducteur du camion-toupie, au moyen de l'interface 28. Lorsque la validation manuelle est réalisée, le procédé se poursuit à l'étape 214. Si, à l'étape 216, la validation manuelle n'est pas réalisée, le procédé retourne à l'étape 200.

A l'étape 214, la quantité d'eau (Ajout) est ajoutée dans le réservoir 12. Ceci peut être réalisé par la commande de la vanne 40 par le module de traitement 26. Le procédé se poursuit à l'étape 218.

A l'étape 218, le procédé attend pendant une durée déterminée, par exemple 5 minutes, pour que l'eau ajoutée se mélange de façon convenable au béton, avant de revenir à l'étape 200.

Le procédé peut, en outre, comprendre l'affichage sur l'écran d'affichage 28 d'informations relatives au béton, l'impression de ces informations sur un support ou le stockage de ces informations dans une mémoire. Ces informations peuvent comprendre le paramètre d'ouvrabilité déterminé à l'étape 200, la quantité d'eau et/ou d'adjuvant ajoutée au béton à l'étape 214 ou la formulation du béton modifiée après l'ajout de l'eau et/ou de l'adjuvant.

Le procédé de contrôle selon l'invention mis en oeuvre par le module de traitement 26 peut être réalisé par voie matérielle, c'est-à-dire par un circuit électronique dédié. A titre de variante, le procédé de contrôle selon l'invention peut être mis en oeuvre au moins en partie par l'exécution par le module de traitement 26 d'instructions d'un programme d'ordinateur par exemple stocké dans la mémoire 27.

Le procédé de contrôle selon l'invention permet avantageusement de déterminer un paramètre d'ouvrabilité lorsque le béton se trouve dans le malaxeur à axe de rotation non-vertical. Il permet, en outre, d'obtenir une mesure du paramètre d'ouvrabilité qui est plus représentative de l'état du béton que la mesure qui serait obtenue à partir d'un test mettant en oeuvre un prélèvement d'un volume faible du béton par rapport au volume total contenu dans le malaxeur à axe de rotation non-vertical.

Des exemples de réalisation particuliers de la présente invention ont été décrits. Diverses variantes et modifications apparaîtront à l'homme de l'art. En particulier, bien que la présente invention ait été décrite dans le cas où le couple moteur est déterminé à partir de mesures de pression hydraulique, il est clair que la présente invention peut être mise en oeuvre dans le cas où le couple moteur est mesuré directement par un capteur de couple, comprenant par exemple des jauges de contraintes. En outre, bien que la présente invention ait été décrite dans le cas d'un malaxeur à axe de rotation non-vertical dont le réservoir est entraîné en rotation par un moteur hydraulique, elle peut être mise en oeuvre dans le cas où le réservoir est entraîné en rotation par un moteur thermique ou par un moteur électrique par l'intermédiaire d'un système mécanique de réduction de vitesse. Le couple moteur peut alors être mesuré par tout moyen adapté. En particulier, lorsque le réservoir est entraîné en rotation par un moteur électrique, le couple moteur peut être déterminé à partir d'une mesure du courant d'alimentation du moteur électrique.

**Revendications**

1. Procédé de contrôle d'au moins un paramètre d'ouvrabilité d'un béton (14) contenu dans le réservoir (12) d'un malaxeur (11) à axe de rotation non-vertical, comprenant les étapes suivantes :

   faire tourner le réservoir à au moins deux vitesses de rotation différentes ;
   déterminer, pour chacune desdites au moins deux vitesses de rotation $\omega$, à partir d'une mesure d'une donnée représentative du couple d'entraînement en rotation du réservoir, un couple C d'entraînement en rotation du réservoir ;
   déterminer, pour chacune desdites au moins deux vitesses de rotation $\omega$, une valeur de contrainte de cisaillement $\tau$ du béton et une valeur de gradient de vitesse $\dot{\gamma}$ du béton selon les relations suivantes :

   $$\tau = T(\omega).C$$
   $$\dot{\gamma} = G(\omega).\omega$$

   où T et G sont des fonctions prédéterminées ;
   déterminer une relation d'évolution de la contrainte de cisaillement $\tau$ en fonction du gradient de vitesse $\dot{\gamma}$ par extrapolation et/ou approximation à partir des valeurs déterminées ; et
   fournir une indication du paramètre d'ouvrabilité du béton à partir de la relation d'évolution.

2. Procédé selon la revendication 1, comprenant les étapes suivantes ;
   faire tourner le réservoir (12) à une première vitesse de rotation et déterminer un premier couple d'entraînement en rotation du réservoir à la première vitesse de rotation ;
   faire tourner le réservoir à une deuxième vitesse de rotation et déterminer un deuxième couple d'entraînement en rotation du réservoir à la deuxième vitesse de rotation ;
   déterminer une première contrainte de cisaillement égale au produit du premier couple et de la valeur de la fonction T à la première vitesse de rotation ;
   déterminer un premier gradient de vitesse égal au produit de la première vitesse de rotation et de la valeur de la fonction G à la première vitesse de rotation ;
   déterminer une deuxième contrainte de cisaillement égale au produit du deuxième couple et de la valeur de la fonction T à la deuxième vitesse de rotation ;
   déterminer un deuxième gradient de vitesse égal au produit de la deuxième vitesse de rotation et de la valeur de la fonction G à la deuxième vitesse de rotation ; et
   déterminer la relation d'évolution de la contrainte de cisaillement en fonction du gradient de vitesse par extrapolation et/ou approximation à partir des première et deuxième contraintes de cisaillement et des premier et deuxième gradients de vitesse.

3. Procédé selon la revendication 2, comprenant les étapes suivantes :

   faire tourner le réservoir (12) à une troisième vitesse de rotation et déterminer un troisième couple d'entraînement en rotation du réservoir à la troisième vitesse de rotation ;
   déterminer une troisième contrainte de cisaillement égale au produit du troisième couple et de la valeur de la fonction T à la troisième vitesse de rotation ;
   déterminer un troisième gradient de vitesse égal au produit de la troisième vitesse de rotation et de la valeur de la fonction G à la troisième vitesse de rotation ; et
   déterminer la relation d'évolution de la contrainte de cisaillement en fonction du gradient de vitesse par extrapolation et/ou approximation en outre à partir de la troisième contrainte de cisaillement et du troisième gradient de vitesse.

4. Procédé selon l'une quelconque des revendications 1 à 3, dans lequel le paramètre d'ouvrabilité du béton est choisi parmi l'affaissement, l'étalement, la contrainte seuil, la viscosité et le temps d'écoulement.

5. Procédé selon l'une quelconque des revendications 1 à 4, comprenant la détermination de la contrainte seuil du béton à partir de la relation d'évolution et la détermination de l'affaissement et/ou de l'étalement à partir de la contrainte seuil.

6. Procédé selon l'une quelconque des revendications 1 à 5, comprenant l'ajustement dans le réservoir du paramètre d'ouvrabilité du béton par l'introduction d'un composé dans le réservoir (12).

**7.** Procédé selon la revendication 6, dans lequel le composé comprend de l'eau, un adjuvant ou un mélange de ceux-ci.

**8.** Procédé selon l'une quelconque des revendications 1 à 7, dans lequel la fourniture de l'indication du paramètre d'ouvrabilité du béton comprend l'affichage sur un écran d'affichage (28) du paramètre d'ouvrabilité, l'impression du paramètre d'ouvrabilité sur un support et/ou le stockage d'une donnée représentative du paramètre d'ouvrabilité dans une mémoire.

**9.** Procédé selon l'une quelconque des revendications 1 à 8, dans lequel le réservoir (12) est entraîné en rotation par un moteur hydraulique (16) comprenant une entrée de réception d'un fluide hydraulique et une sortie de refoulement du fluide hydraulique, le couple étant déterminé à partir d'une première différence de pressions égale à la différence entre la pression hydraulique mesurée à l'entrée du moteur hydraulique et la pression hydraulique mesurée à la sortie du moteur hydraulique.

**10.** Procédé selon la revendication 9, dans lequel la première différence de pressions est diminuée d'une deuxième différence de pressions égale à la différence entre la pression hydraulique à l'entrée du moteur hydraulique (16) et la pression hydraulique à la sortie du moteur hydraulique en l'absence de béton dans le réservoir (12) à la vitesse de rotation de la mesure.

**11.** Procédé selon la revendication 9 ou 10, dans lequel la pression hydraulique mesurée à l'entrée ou à la sortie du moteur hydraulique (16) est égale à la moyenne d'un nombre de valeurs de pression échantillonnées, ledit nombre étant inversement proportionnel à la vitesse de rotation du réservoir (12).

**12.** Procédé selon la revendication 11, dans lequel pendant l'échantillonnage des valeurs de pression utilisées pour obtenir la pression hydraulique mesurée à l'entrée ou à la sortie du moteur hydraulique (16), les variations de la vitesse de rotation du réservoir (12) sont inférieures à un seuil.

**13.** Procédé selon l'une quelconque des revendications 1 à 12, dans lequel les fonctions G et T sont obtenues en déterminant :

pour chaque béton d'une pluralité de bétons différents, une courbe d'évolution du couple d'entraînement du réservoir (12) contenant ledit béton en fonction de la vitesse de rotation du réservoir ;
pour chaque béton de la pluralité de bétons différents, une courbe d'évolution de la contrainte de cisaillement du béton en fonction du gradient de vitesse du béton au moyen d'un rhéomètre ; et
pour chaque paire de bétons de la pluralité de bétons différents, un premier point d'intersection ($H_i$) entre les courbes d'évolution du couple d'entraînement du réservoir en fonction de la vitesse de rotation du réservoir pour les bétons de la paire et un deuxième point d'intersection ($L_i$) entre les courbes d'évolution de la contrainte de cisaillement en fonction du gradient de vitesse pour les bétons de la paire.

**14.** Procédé selon la revendication 13, dans lequel pour le premier point d'intersection ($H_i$) et le deuxième point d'intersection ($L_i$) de chaque paire de bétons de la pluralité de bétons différents, il est déterminé une valeur $G_i^{CC}$ de la fonction G et une valeur $T_i^{CC}$ de la fonction T selon les relations suivantes :

$$G_i^{CC} = \dot{\gamma}_i/\omega_i$$
$$T_i^{CC} = \tau_i/C_i$$

où $\dot{\gamma}_i$ est le gradient de vitesse au second point d'intersection, $\tau_i$ est la contrainte de cisaillement du béton au second point d'intersection, $C_i$ est le couple d'entraînement au premier point d'intersection et $\omega_i$ est la vitesse de rotation au premier point d'intersection.

**15.** Procédé selon la revendication 13, dans lequel pour le premier point d'intersection ($H_i$) et le second point d'intersection ($L_i$) de chaque paire de bétons de la pluralité de bétons différents, il est déterminé une valeur $G_i^{Alt}$ de la fonction G et une valeur $T_i^{Alt}$ de la fonction T selon les relations suivantes :

$$G_i^{Alt} = \sqrt{\frac{C_i}{V \cdot \eta_i \cdot \omega_i}}$$

$$T_i^{Alt} = \frac{1}{G_i^{Alt} \cdot V}$$

où V est le volume du béton (14) dans le réservoir (12), $\eta_i$ est la viscosité apparente du béton égale au rapport entre la contrainte de cisaillement du béton au second point d'intersection ($L_i$) et le gradient de vitesse au second point d'intersection, $C_i$ est le couple d'entraînement au premier point d'intersection ($H_i$) et $\omega_i$ est la vitesse de rotation au premier point d'intersection.

16. Dispositif mémoire sur lequel est stocké un programme d'ordinateur pour la mise en oeuvre du procédé selon l'une quelconque des revendications 1 à 15.

17. Dispositif (10) de contrôle d'au moins un paramètre d'ouvrabilité d'un béton, comprenant :

un malaxeur à axe de rotation non-vertical (11) comportant un réservoir (12) contenant le béton (14) ;
un système d'entraînement (16, 18) en rotation du réservoir adapté à faire tourner le réservoir à au moins deux vitesses de rotation différentes ;
un premier capteur (30, 32) de mesure d'une donnée représentative du couple d'entraînement en rotation du réservoir ;
un deuxième capteur (34, 35) de mesure d'une donnée représentative de la vitesse de rotation du réservoir ; et
un module de traitement (26) relié au système d'entraînement et aux premier et deuxième capteurs et configuré pour
déterminer, pour chacune desdites au moins deux vitesses de rotation $\omega$, à partir de la donnée représentative du couple d'entraînement en rotation du réservoir, un couple d'entrainement C en rotation du réservoir, et
déterminer, pour chacune desdites au moins deux vitesses de rotation $\omega$, une valeur de contrainte de cisaillement $\tau$ du béton et une valeur de gradient de vitesse $\dot{\gamma}$ du béton selon les relations suivantes :

$$\tau = T(\omega) \cdot C$$

$$\dot{\gamma} = G(\omega) \cdot \omega$$

où T et G sont des fonctions prédéterminées ; déterminer une relation d'évolution de la contrainte de cisaillement $\tau$ en fonction du gradient de vitesse $\dot{\gamma}$ par extrapolation et/ou approximation à partir des valeurs déterminées ; et fournir une indication du paramètre d'ouvrabilité du béton à partir de la relation d'évolution.

**Patentansprüche**

1. Verfahren zum Kontrollieren mindestens eines Verarbeitbarkeitsparameters eines Betons (14), der in dem Behälter (12) eines Mischers (11) mit einer nicht-vertikalen Drehachse enthalten ist, umfassend die folgenden Schritte:

Veranlassen, dass sich der Behälter bei mindestens zwei verschiedenen Drehgeschwindigkeiten dreht;
Bestimmen eines Drehantriebsdrehmoments C des Behälters für jede der mindestens zwei Drehgeschwindigkeiten $\omega$ auf Basis einer Messung von Daten, die für das Drehantriebsdrehmoment des Behälters repräsentativ sind,
Bestimmen, für jede der mindestens zwei Drehgeschwindigkeiten $\omega$, eines Wert der Schubspannung $\tau$ des Betons und eines Werts des Geschwindigkeitsgefälles $\dot{\gamma}$ des Betons gemäß den folgenden Beziehungen:

$$\tau = T(\omega) \cdot C$$

$$\dot{\gamma} = G(\omega) \cdot \omega$$

wobei T und G vorbestimmte Funktionen sind;

Bestimmen einer Variationsbeziehung der Schubspannung $\tau$ gemäß dem Geschwindigkeitsgefälle $\dot{\gamma}$ durch Extrapolation und/oder Näherung auf Basis der bestimmten Werte; und

Bereitstellen einer Anzeige des Verarbeitbarkeitsparameters des Betons auf Basis der Variationsbeziehung.

2. Verfahren nach Anspruch 1, umfassend die folgenden Schritte:

Veranlassen, dass sich der Behälter (12) bei einer ersten Drehgeschwindigkeit dreht und Bestimmen eines ersten Drehantriebsdrehmoments des Behälters bei der ersten Drehgeschwindigkeit;

Veranlassen, dass sich der Behälter bei einer zweiten Drehgeschwindigkeit dreht und Bestimmen eines zweiten Drehantriebsdrehmoments des Behälters bei der zweiten Drehgeschwindigkeit;

Bestimmen einer ersten Schubspannung entsprechend dem Produkt des ersten Drehmoments und des Wertes der Funktion T bei der ersten Drehgeschwindigkeit;

Bestimmen eines ersten Geschwindigkeitsgefälles entsprechend dem Produkt der ersten Drehgeschwindigkeit und des Wertes der Funktion G bei der ersten Drehgeschwindigkeit;

Bestimmen einer zweiten Schubspannung entsprechend dem Produkt des zweiten Drehmoments und des Wertes der Funktion T bei der zweiten Drehgeschwindigkeit;

Bestimmen eines zweiten Geschwindigkeitsgefälles entsprechend dem Produkt der zweiten Drehgeschwindigkeit und des Wertes der Funktion G bei der zweiten Drehgeschwindigkeit; und

Bestimmen der Variationsbeziehung der Schubspannung gemäß dem Geschwindigkeitsgefälle durch Extrapolation und/oder Näherung auf Basis der ersten und zweiten Schubspannung und des ersten und zweiten Geschwindigkeitsgefälles.

3. Verfahren nach Anspruch 2, umfassend die folgenden Schritte:

Veranlassen, dass sich der Behälter (12) bei einer dritten Drehgeschwindigkeit dreht und Bestimmen eines dritten Drehantriebsdrehmoments des Behälters bei der dritten Drehgeschwindigkeit;

Bestimmen einer dritten Schubspannung entsprechend dem Produkt des dritten Drehmoments und des Wertes der Funktion T bei der dritten Drehgeschwindigkeit;

Bestimmen eines dritten Geschwindigkeitsgefälles entsprechend dem Produkt der dritten Drehgeschwindigkeit und des Wertes der Funktion G bei der dritten Drehgeschwindigkeit; und

Bestimmen der Variationsbeziehung der Schubspannung gemäß dem Geschwindigkeitsgefälle durch Extrapolation und/oder Näherung zusätzlich auf Basis der dritten Schubspannung und des dritten Geschwindigkeitsgefälles.

4. Verfahren nach einem der Ansprüche 1 bis 3, bei dem der Verarbeitbarkeitsparameter des Betons aus dem Setzmaß, dem Fließmaß, der Schwellenwert-Beanspruchung, der Viskosität und der Fließgeschwindigkeit ausgewählt ist.

5. Verfahren nach einem der Ansprüche 1 bis 4, umfassend die Bestimmung der Schwellenwert-Beanspruchung des Betons auf Basis der Variationsbeziehung und die Bestimmung des Setzmaßes und/oder Fließmaßes auf Basis der Schwellenwert-Beanspruchung.

6. Verfahren nach einem der Ansprüche 1 bis 5, umfassend die in dem Behälter erfolgende Anpassung des Verarbeitbarkeitsparameters des Betons durch Einführen einer Verbindung in den Behälter (12).

7. Verfahren nach Anspruch 6, bei dem die Verbindung Wasser, ein Adjuvans oder ein Gemisch davon umfasst.

8. Verfahren nach einem der Ansprüche 1 bis 7, bei dem das Bereitstellen der Anzeige des Verarbeitbarkeitsparameters des Betons die Anzeige des Verarbeitbarkeitspaxameters auf einem Bildschirm (28), das Ausdrucken des Verarbeitbarkeitsparameters auf einem Träger und/oder die Speicherung einer Dateneinheit, die den Verarbeitbarkeitsparameter repräsentiert, in einem Datenspeicher beinhaltet.

9. Verfahren nach einem der Ansprüche 1 bis 8, bei dem der Behälter (12) in Rotation durch einen hydraulischen Motor (16) angetrieben wird, der einen Einlass zum Empfangen eines hydraulischen Fluids und einen Auslass zum Zurückdrängen des hydraulischen Fluids umfasst, wobei das Drehmoment auf Basis einer ersten Druckdifferenz, entsprechend der Differenz zwischen dem hydraulischen Druck, der am Einlass des hydraulischen Motors gemessen wird, und dem hydraulischen Druck, der am Auslass des hydraulischen Motors gemessen wird, bestimmt wird.

10. Verfahren nach Anspruch 9, bei dem die erste Druckdifferenz um eine zweite Druckdifferenz entsprechend der Differenz zwischen dem hydraulischen Druck am Einlass des hydraulischen Motors (16) und dem hydraulischen Druck am Auslass des hydraulischen Motors in Abwesenheit von Beton in dem Behälter (12) bei der Drehgeschwindigkeitsmessung verringert wird.

11. Verfahren nach Anspruch 9 oder 10, bei dem der hydraulische Druck, der am Einlass oder am Auslass des hydraulischen Motors (16) gemessen wird, dem Mittelwert einer Anzahl abgefragter Druckwerte entspricht, wobei diese Zahl zu der Drehgeschwindigkeit des Behälters (12) umgekehrt proportional ist.

12. Verfahren nach Anspruch 11, bei dem während des Abfragens der Druckwerte, die zum Erhalten des hydraulischen Drucks, gemessen am Einlass oder am Auslass des hydraulischen Motors (16), verwendet werden, die Variationen der Drehgeschwindigkeit des Behälters (12) niedriger sind als ein Schwellenwert.

13. Verfahren nach einem der Ansprüche 1 bis 12, bei dem die Funktionen G und T erhalten werden, indem man folgendes bestimmt:

für jeden Beton von einer Mehrzahl verschiedener Betons, eine Variationskurve des Antriebsdrehmoments des den Beton enthaltenden Behälters (12) gemäß der Drehgeschwindigkeit des Behälters;
für jeden Beton von einer Mehrzahl verschiedener Betons, eine Variationskurve der Schubspannung des Betons gemäß dem Geschwindigkeitsgefälle des Betons mittels eines Rheometers; und
für jedes Paar von Betons der Mehrzahl verschiedener Betons, einen ersten Schnittpunkt ($H_i$) zwischen den Variationskurven des Antriebsdrehmoments des Behälters gemäß der Drehgeschwindigkeit des Behälters für die Betons des Paares und einen zweiten Schnittpunkt ($L_i$) zwischen den Variationskurven der Schubspannung gemäß dem Geschwindigkeitsgefälle für die Betons des Paares.

14. Verfahren nach Anspruch 13, bei dem für den ersten Schnittpunkt ($H_i$) und den zweiten Schnittpunkt ($L_i$) jedes Paares von Betons der Mehrzahl verschiedener Betons, der Wert $Gi^{CC}$ der Funktion G und der Wert $Ti^{cc}$ der Funktion T gemäß den folgenden Beziehungen bestimmt wird;

$$G_i^{CC} = \frac{\dot{\gamma}_i}{\omega_i}$$

$$T_i^{CC} = \frac{\tau_i}{C_i}$$

wobei $\dot{\gamma}_i$ das Geschwindigkeitsgefälle am zweiten Schnittpunkt ist, $t_i$ die Schubspannung des Betons am zweiten Schnittpunkt ist, $C_i$ das Antriebsdrehmoment am ersten Schnittpunkt ist und $\omega_i$ die Drehgeschwindigkeit am ersten Schnittpunkt ist.

15. Verfahren nach Anspruch 13, bei dem für den ersten Schnittpunkt ($H_i$) und den zweiten Schnittpunkt ($L_i$) jedes Paares von Betons der Mehrzahl verschiedener Betons, ein Wert $Gi^{Alt}$ der Funktion G und ein Wert $Ti^{Alt}$ der Funktion T gemäß den folgenden Beziehungen bestimmt wird:

$$G_i^{Alt} = \sqrt{\frac{C_i}{V \cdot \eta_i \cdot \omega_i}}$$

$$T_i^{Alt} = \frac{1}{G_i^{Alt} \cdot V}$$

wobei $v$ das Volumen des Betons (14) in dem Behälter (12) ist, $\eta_i$ die scheinbare Viskosität des Betons entsprechend dem Verhältnis der Schubspannung des Betons am zweiten Schnittpunkt ($L_i$) und des Geschwindigkeitsgefälles am zweiten Schnittpunkt ist, $C_i$ das Antriebsdrehmoment am ersten Schnittpunkt ($H_i$) ist und $\omega_i$ die Drehgeschwindigkeit am ersten Schnittpunkt ist.

16. Speichervorrichtung, in der ein Computerprogramm zum Implementieren des Verfahrens nach einem der Ansprüche 1 bis 15 gespeichert ist.

17. Vorrichtung (10) zum Kontrollieren mindestens eines Verarbeitbarkeitsparameters eines Betons, umfassend:

einen Mischer mit einer nicht-vertikalen Drehachse (11), umfassend einen Behälter (12), der den Beton (14) enthält;

ein System (16, 18), um den Behälter in Rotation anzutreiben, geeignet, um zu veranlassen, dass sich der Behälter bei mindestens zwei verschiedenen Drehgeschwindigkeiten dreht;

einen ersten Sensor (30, 32) zum Messen einer Dateneinheit, die das Drehantriebsdrehmoment des Behälters repräsentiert;

einen zweiten Sensor (34, 35) zum Messen einer Dateneinheit, die die Drehgeschwindigkeit des Behälters repräsentiert; und

ein Verarbeitungsmodul (26), verbunden mit dem Antriebssystem und mit den ersten und zweiten Sensoren, und konfiguriert um

für jede der mindestens zwei Drehgeschwindigkeiten $\omega$, auf Basis einer Messung von Daten, die für das Drehantriebsdrehmoment des Behälters repräsentativ sind, ein Drehantriebsdrehmoment C des Behälters zu bestimmen,

für jede der mindestens zwei Drehgeschwindigkeiten $\omega$ einen Wert für die Schubspannung $\tau$ des Betons und einen Wert für das Geschwindigkeitsgefälle $\dot{\gamma}$ des Betons gemäß den folgenden Beziehungen zu bestimmen:

$$\tau = T(\omega) \cdot C$$

$$\dot{\gamma} = G(\omega) \cdot \omega$$

wobei T und G vorbestimmte Funktionen sind;

eine Variationsbeziehung der Schubspannung $\tau$ gemäß dem Geschwindigkeitsgefälle $\dot{\gamma}$ durch Extrapolation und/oder Näherung auf Basis der vorab ermittelten Werte zu bestimmen; und

eine Anzeige des Verarbeitbarkeitsparameters des Betons auf Basis der Variationsbeziehung bereitzustellen.

**Claims**

1. A method for controlling at least one workability parameter of a concrete (14) contained in the container (12) of a mixer (11) with a non vertical rotational axis, comprising the following steps:

making the container turn at at least two different rotational speeds;

determining, for each of said at least two rotational speeds $\omega$, based on a measurement of data representative of the rotary drive torque of the container, a rotary drive torque C of the container,

determining, for each of said at least two rotational speeds $\omega$, a value of shear stress $\tau$ of the concrete and a value of speed gradient $\dot{\gamma}$ of the concrete according to the following relationships:

$$\tau = T(\omega) \cdot C$$

$$\dot{\gamma} = G(\omega) \cdot \omega$$

where T and G are predetermined functions;

determining a relationship of variation of the shear stress $\tau$ according to the speed gradient $\dot{\gamma}$ by extrapolation and/or approximation based on the determined values; and

providing an indication of the workability parameter of the concrete based on the relationship of variation.

2. The method according to claim 1, comprising the following steps:

making the container (12) turn at a first rotational speed and determining a first rotary drive torque of the container at the first rotational speed;

making the container turn at a second rotational speed and determining a second rotary drive torque of the container at the second rotational speed;

determining a first shear stress equal to the product of the first torque and to the value of the function T at the first rotational speed;

determining a first speed gradient equal to the product of the first rotational speed and to the value of the function G at the first rotational speed;

determining a second shear stress equal to the product of the second torque and to the value of the function T at the second rotational speed;

determining a second speed gradient equal to the product of the second rotational speed and to the value of the function G at the second rotational speed; and

determining the relationship of variation of the shear stress according to the speed gradient by extrapolation and/or approximation based on the first and second shear stresses and the first and second speed gradients.

3. The method according to claim 2, comprising the following steps:

making the container (12) turn at a third rotational speed and determining a third rotary drive torque of the container at the third rotational speed;

determining a third shear stress equal to the product of the third torque and to the value of the function T at the third rotational speed;

determining a third speed gradient equal to the product of the third rotational speed and to the value of the function G at the third rotational speed; and

determining the relationship of variation of the shear stress according to the speed gradient by extrapolation and/or approximation in addition based on the third shear stress and the third speed gradient.

4. The method according to any one of claims 1 to 3, in which the workability parameter of the concrete is selected from among the slump, the slump flow, the threshold stress, the viscosity and the flow rate.

5. The method according to any one of claims 1 to 4, comprising the determination of the threshold stress of the concrete based on the relationship of variation and the determination of the slump and/or slump flow based on the threshold stress.

6. The method according to any one of claims 1 to 5, comprising the adjustment in the container of the workability parameter of the concrete by introducing a compound into the container (12).

7. The method according to claim 6, in which the compound comprises water, an adjuvant or a mixture thereof.

8. The method according to any one of claims 1 to 7, in which providing the indication of the workability parameter of the concrete includes the display on a display screen (28) of the workability parameter, the printing out of the workability parameter onto a support and/or the storage of a datum representing the workability parameter to a memory.

9. The method according to any one of claims 1 to 8, in which the container (12) is driven in rotation by a hydraulic motor (16) comprising an inlet for receiving a hydraulic fluid and an outlet for pushing back the hydraulic fluid, the torque being determined based on a first difference of pressures equal to the difference between the hydraulic pressure measured at the inlet of the hydraulic motor and the hydraulic pressure measured at the outlet of the hydraulic motor.

10. The method according to claim 9, in which the first difference of pressures is decreased by a second difference of pressures equal to the difference between the hydraulic pressure at the inlet of the hydraulic motor (16) and the hydraulic pressure at the outlet of the hydraulic pressure in the absence of concrete in the container (12) at the measurement rotational speed.

11. The method according to claim 9 or 10, in which the hydraulic pressure measured at the inlet or at the outlet of the hydraulic motor (16) is equal to the average of a number of sampled pressure values, said number being inversely proportional to the rotational speed of the container (12).

**12.** The method according to claim 11, in which during the sampling of the pressure values used for obtaining the hydraulic pressure measured at the inlet or at the outlet of the hydraulic motor (16), the variations of the rotational speed of the container (12) are lower than a threshold.

**13.** The method according to any one of claims 1 to 12, in which the functions G and T are obtained by determining:

for each concrete of a plurality of different concretes, a variation curve of the drive torque of the container (12) containing said concrete according to the rotational speed of the container;
for each concrete of a plurality of different concretes, a variation curve of the shear stress of the concrete according to the speed gradient of the concrete by means of a rheometer; and
for each pair of concretes of the plurality of different concretes, a first point of intersection ($H_i$) between the variation curves of the drive torque of the container according to the rotational speed of the container for the concretes of the pair and a second point of intersection ($L_i$) between the variation curves of the shear stress according to the speed gradient for the concretes of the pair.

**14.** The method according to claim 13, in which for the first point of intersection ($H_i$) and the second point of intersection ($L_i$) of each pair of concretes of the plurality of different concretes, it is determined the value $G_i^{cc}$ of the function G and the value $T_i^{cc}$ of the function T according to the following relationships:

$$G_i^{CC} = \frac{\dot{\gamma}_i}{\omega_i}$$

$$T_i^{CC} = \frac{\tau_i}{C_i}$$

where $\dot{\gamma}_i$ is the speed gradient at the second point of intersection, $t_i$ is the shear stress of the concrete at the second point of intersection, $C_i$ is the drive torque at the first point of intersection and $\omega_i$ is the rotational speed at the first point of intersection.

**15.** The method according to claim 13, in which for the first point of intersection ($H_i$) and the second point of intersection ($L_i$) of each pair of concretes of the plurality of different concretes, it is determined a value $G_i^{Alt}$ of the function G and a value $T_i^{Alt}$ of the function T according to the following relationships:

$$G_i^{Alt} = \sqrt{\frac{C_i}{V \cdot \eta_i \cdot \omega_i}}$$

$$T_i^{Alt} = \frac{1}{G_i^{Alt} \cdot V}$$

where V is the volume of concrete (14) in the container (12), $\eta_i$ is the apparent viscosity of the concrete equal to the ratio of the shear stress of the concrete at the second point of intersection ($L_i$) and the speed gradient at the second point of intersection, $C_i$ is the drive torque at the first point of intersection ($H_i$) and $\omega_i$ is the rotational speed at the first point of intersection.

**16.** A memory device on which is stored a computer programme for implementing the method according to any one of claims 1 to 15.

**17.** A device (10) for controlling at least one workability parameter of a concrete, comprising:

a mixer with a non vertical rotational axis (11) comprising a container (12) containing the concrete (14);
a system (16, 18) for driving in rotation the container suited for making the container turn at at least two different rotational speeds;
a first sensor (30, 32) for measuring a datum representing the rotary drive torque of the container;

a second sensor (34, 35) for measuring a datum representing the rotational speed of the container; and
a processing module (26) connected to the drive system and to the first and second sensors and configured to determine, for each of said at least two rotational speeds $\omega$, based on a measurement of data representative of the rotary drive torque of the container, a rotary drive torque C of the container,
determining, for each of said at least two rotational speeds $\omega$, a value of shear stress $\tau$ of the concrete and a value of speed gradient $\dot{\gamma}$ of the concrete according to the following relationships:

$$\tau = T(\omega) \cdot C$$

$$\dot{\gamma} = G(\omega) \cdot \omega$$

where T and G are predetermined functions;
determine, a relationship of variation of the shear stress $\tau$ according to the speed gradient $\dot{\gamma}$ by extrapolation and/or approximation based on the predetermined values; and
provide an indication of the workability parameter of the concrete based on the relationship of variation.

Fig 1

Fig 2

Fig 3

Fig 4

Fig 5

Fig 6

Fig 7

Fig 8

Fig 9

25

$S_k$ — 200

$\Delta S \geqslant TH$ ? — 202

N

O

Détermination Ajout — 204

206

$Eau_{tot} \leqslant Max$ ? — 208

N → Alarme

O

212

Ajout automatique ? — 216

N → Validation Manuelle ?

N

O

O

Ajout d'Eau — 214

Attente Stabilisation — 218

Fig 10

**EP 2 831 562 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 5713663 A